# EUROPÄISCHE PATENTSCHRIFT

(11) **EP 0 780 094 B1**
(45) Veröffentlichungstag und Bekanntmachung des Hinweises auf die Patenterteilung: **21.11.2001**
(21) Anmeldenummer: 96119548.4
(22) Anmeldetag: 05.12.1996
(51) Int. Cl.: A61B 17/68, A61B 17/58

(54) **Osteosynthesevorrichtung**
Osteosynthesis device
Dispositif d'ostéosynthèse

(30) Priorität: 22.12.1995 DE 19548395
(43) Veröffentlichungstag der Anmeldung: 25.06.1997
(73) Patentinhaber: Stryker Leibinger GmbH & Co. KG, 79111 Freiburg (DE)
(72) Erfinder: Raveh, Joram, Prof. Dr. Dr., CH-3097 Liebefeld-Bern (CH)
(74) Vertreter: von Hellfeld, Axel, Dr. Dipl.-Phys.

(56) Entgegenhaltungen:
- EP-A- 0 048 038
- EP-A- 0 201 024
- CH-A- 675 531

## Beschreibung

Die Erfindung betrifft eine Osteosynthesevorrichtung aus einer Platte mit einer oberen Oberfläche und einer gegenüberliegenden, einem Knochen zugekehrten unteren Oberfläche sowie einer Mehrzahl von in der Platte ausgeformten Schraubenlöchern, deren Durchmesser an der oberen Oberfläche der Platte größer ist als ihr Durchmesser an der unteren Plattenoberfläche, und mit durch die Schraubenlöcher in den Knochen einzuschraubenden Schrauben, die einen zumindest teilweise gerundeten Kopf haben, der in eingeschraubtem Zustand mit einer Kontaktfläche an der Wand des Schraubenloches anliegt.

Eine Osteosynthesevorrichtung ist aus der CH 675 531 A5 bekannt. Die dort beschriebene Fixationsvorrichtung für die Osteosynthese soll eine rigide, funktionsstabile Fixation (örtliche Positionierung) zwischen dem Kopf der Knochenschraube und der Osteosyntheseplatte gewährleisten. Im Stand der Technik gemäß der CH 675 531 A5 liegt die Berührungslinie zwischen sphärischem Schraubenkopf und konischem Schraubenloch etwa mittig zwischen der unteren und der oberen Oberfläche der Osteosyntheseplatte. Das konische Plattenloch und der Schraubenkopf sind so konzipiert, daß es beim Anziehen der Schrauben zum Durchstoßen des Schraubenkopfes durch die Platten kommen kann. Somit wird eine stabile Fixation des Schraubenkopfes an die Platte nicht gewährleistet. Ein solches System kann nicht die Anforderungen erfüllen.

Insbesondere wird mit der vorliegenden Erfindung eine Vereinigung der Vorteile der bekannten externen Fixierung ("fixateur externe") mit den Vorteilen der bekannten internen Plattenbefestigung ("fixateur interne") angestrebt, um eine langfristig funktionsstabile Verankerung des Implantates zu erreichen. Der Stand der Technik erreicht weitgehend die genannten Ziele mit Hilfe eines spreizbaren Schraubenkopfes und vermeidet damit eine Knochenresorption an der Auflagefläche zwischen der Platte und dem Knochen. Eine derartige Knochenresorption ist besonders aufgrund der ihr in der Regel nachfolgenden Instabilität und Lockerung der Fixation zu vermeiden. Jedoch können mit solch einer Vorrichtung die Schrauben nur in einem rechten Winkel zur Platte eingebracht und in den Knochen eingesetzt werden.

Der Erfindung liegt die Aufgabe zugrunde, die Osteosynthesevorrichtung weiter mit Blick auf die genannten Ziele zu verbessern, also insbesondere die langfristige Funktionsstabilität weiter zu verbessern und Lockerungen durch Spannungen und Belastungen, die auf Knochen und Platte bei funktionalen Bewegungen übertragen werden, weitestgehend auszuschließen.

Die Erfindung löst diese Aufgabe dadurch, daß bei senkrecht zur Platte stehender Schraube die Kontaktfläche näher an der oberen als an der unteren Oberfläche der Platte liegt.

Durch das neue Konstruktionsprinzip wird eine stabile Verkeilung und Fixation des Schraubenkopfes an der Platte erreicht und gewährleistet, ohne daß ein spreizbarer Schraubenkopf benötigt wird. Der Kontakt findet oberhalb der Mittellinie des Plattenloches statt und somit wird das Durchstossen des Schraubenkopfes vermieden, wobei auch bei der Winkeleinstellung eine stabile bandförmige Fixation und Verkeilung des Schraubenkopfes im gesamten Plattenlochbereich erzielt wird.

Bevorzugt liegt die Kontaktfläche an allen Stellen näher an der oberen, vom Knochen abgekehrten Oberfläche der Osteosyntheseplatte als an der unteren Oberfläche. Es hat sich überraschend herausgestellt, daß durch diese Maßnahme eine Verbesserung im Sinne der oben gestellten Aufgabe erreicht wird. Darüber hinaus sichert eine derartige Positionierung der Kontaktfläche zwischen Schraubenkopf und Schraubenloch die Schraube optimal gegen ein Durchrutschen durch das Loch bei starken Zugspannungen.

Die erfindungsgemäße Osteosynthesevorrichtung ermöglicht, im Gegensatz zu den meisten Vorrichtungen dieser Art des Standes der Technik, daß eine feste interne Fixierung von Knochenfragmenten möglich ist, ohne daß die Platte direkt an den Knochen angepaßt werden muß. Hierdurch wird ein starker Druck auf die Knochenoberfläche und die damit einhergehende Gefahr einer Knochenresorption und Osteolyse vermieden. Dies ist insbesondere bedeutsam für die Fixation (örtliche Festlegung) von konvexen oder konkaven Knochenfragmenten, bei denen keine oder nur ganz geringe Kontakte zur unteren Oberfläche der Platte bestehen.

Die Erfindung gewährleistet eine rigide und langzeitstabile Fixierung des Schraubenkopfes im Loch der Osteosyntheseplatte, so daß Kippbewegungen unmöglich werden und auch eine Lockerung der Schraube, insbesondere infolge einer Knochenresorption im Bereich des Gewindes, ausgeschlossen ist. Hierdurch wird insbesondere die Fixierung von Knochenfragmenten verbessert.

Die Erfindung ermöglicht eine starke axiale Neigung der Schraube in Bezug auf die Plattennormale, wodurch schräg liegende und sagittale Brüche und Knochenfragmente als auch konvexe und konkave Knochenoberflächen fixierbar sind.

Eine bevorzugte Ausgestaltung der Erfindung sieht vor, daß der gerundete, insbesondere sphärische Schraubenkopf im Äquatorbereich hinreichend breit gerundet ist, um in jeder Position eine volle Verkeilung zu gewährleisten.

Gemäß einer weiteren bevorzugten Ausgestaltung werden der gerundete, also ballige, insbesondere sphärische Schraubenkopf und das Schraubenloch so ausgestaltet, daß in jeder möglichen Relativstellung von Schraube und Platte, also insbesondere bei maximal zulässiger Schrägstellung der Schraube in Bezug auf die Platte, die Kontaktfläche zwischen Schraubenkopf und Lochwand vollständig von einem gerundeten Abschnitt des Schraubenkopfes gebildet wird, also nicht von einem isolierten Kantenabschnitt des Schraubenkopfes. Hierdurch wird ein optimaler, breitflächiger Kontakt und eine Verkeilung zwischen Schraubenkopf und Platte gewährleistet, was sich zur Vermeidung von Lockerungen aufgrund von Mikrobewegungen als vorteilhaft herausgestellt hat.

Bevorzugt wird das konusförmige Schraubenloch so gestaltet, daß der Konuswinkel zwischen 3° und 5,5°, besonders bevorzugt zwischen 3,5° und 4,5°, liegt.

Der Erfindungsgedanke läßt sich auch so formulieren, daß der maximale Durchmesser Dₘ des balligen, insbesondere sphärischen Schraubenkopfes größer ist als 1/2 (Dᵤ + Dₒ), wobei Dᵤ und Dₒ die Durchmesser des Schraubenloches in der unteren bzw. oberen Plattenoberfläche sind.

Eine besonders bevorzugte Ausgestaltung der Erfindung sieht vor, daß bei senkrecht zur Platte stehender Schraube der Abstand D_{c} des Berührungsringes von der unteren Oberfläche der Platte im Bereich von 0,6 bis 0,8 der Plattenstärke (dₚ) liegt.

Eine andere bevorzugte Ausgestaltung der Erfindung sieht vor, daß der Schraubenkopf in eingeschraubtem Zustand in dem Schraubenloch versenkt ist.

Nachfolgend werden Ausführungsbeispiele der Erfindung anhand der Zeichnungen näher beschrieben. Es zeigt:
- Fig. 1: ein erstes Ausführungsbeispiel einer Osteosynthesevorrichtung mit Platte und Schraube schematisch im Schnitt;
- Fig. 2: das Ausführungsbeispiel gemäß Fig. 1 mit schräg gestellter Schraube und
- Fig. 3: ein weiteres Ausführungsbeispiel einer Osteosynthesevorrichtung mit Platte und Schraube.

In den Figuren sind einander entsprechende oder funktionsähnliche Bauteile mit gleichen Bezugszeichen versehen.

Die Figuren zeigen eine Platte 1, deren Abmessungen (Dicke etc.) insoweit denen herkömmlicher Osteosynthesevorrichtungen entspricht (vgl. den oben zitierten Stand der Technik). Die Platte 1 hat eine obere Oberfläche 2 und eine untere Oberfläche 3, die im Einsatz dem Knochen zugekehrt ist. Der Abstand zwischen den oberen und unteren Oberflächen ist dₚ.

Mehrere Schraubenlöcher 4 erstrecken sich durch die Platte 4 von der oberen Oberfläche 2 zur unteren Oberfläche 3. Der Durchmesser Dₒ der Schraubenlöcher 4 an der oberen Oberfläche 2 ist größer als der Durchmesser Dᵤ an der unteren Oberfläche 3 der Platte. Die Wand 5 jedes Schraubenloches 4 bildet bei diesem Ausführungsbeispiel einen Konus mit einem Konuswinkel α von 4°.

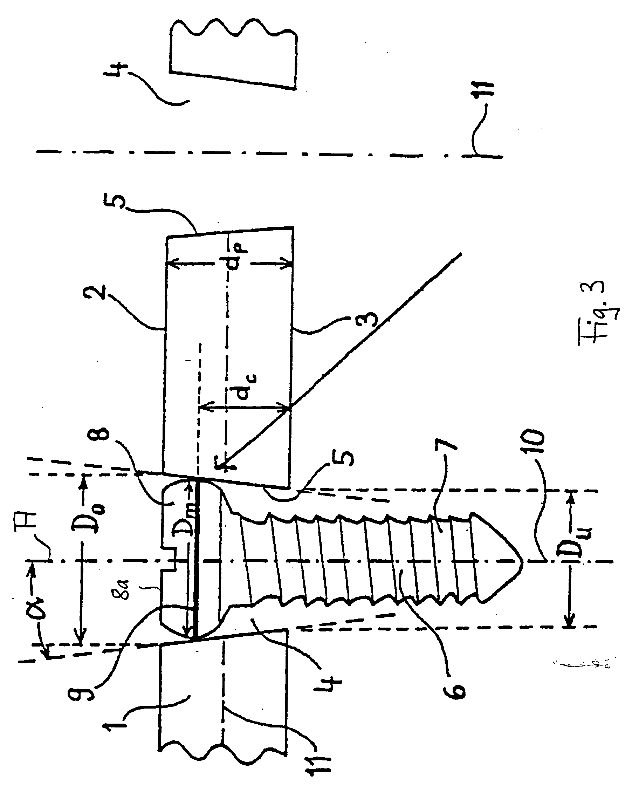

Durch die Löcher 4 werden im Einsatz Schrauben 6 mit Schraubengewinde 7 geführt, wobei der Schraubenkopf 8 jeweils sphärisch ausgeformt ist, z.B. als Kugelabschnitt. Die Schraube wird in bekannter Weise in den Knochen eingeschraubt. Beim Ausführungsbeispiel gemäß Fig. 3 versinkt der Schraubenkopf im wesentlichen im Schraubenloch 4, d.h. bei senkrechter Anordnung der Längsachse A der Schraube 6 in Bezug auf die Hauptebene der Platte 1 bildet die Stirnfläche 8a des Schraubenkopfes 8 mit der oberen Oberfläche 2 der Platte 1 eine im wesentlichen durchgehende Ebene, d.h. die Stirnfläche 8a des Schraubenkopfes 8 fluchtet mit der Oberfläche 2 der Platte 1.

Die fest in den Knochen eingeschraubten Schrauben 6 sind mit ihren Schraubenköpfen 8 fest mit der Wand 5 der Löcher 4 verklemmt. Der sphärische Schraubenkopf 8 und die Wand 5 des Loches stehen in einer bandförmigen Kontaktfläche 9 miteinander in Kontakt.

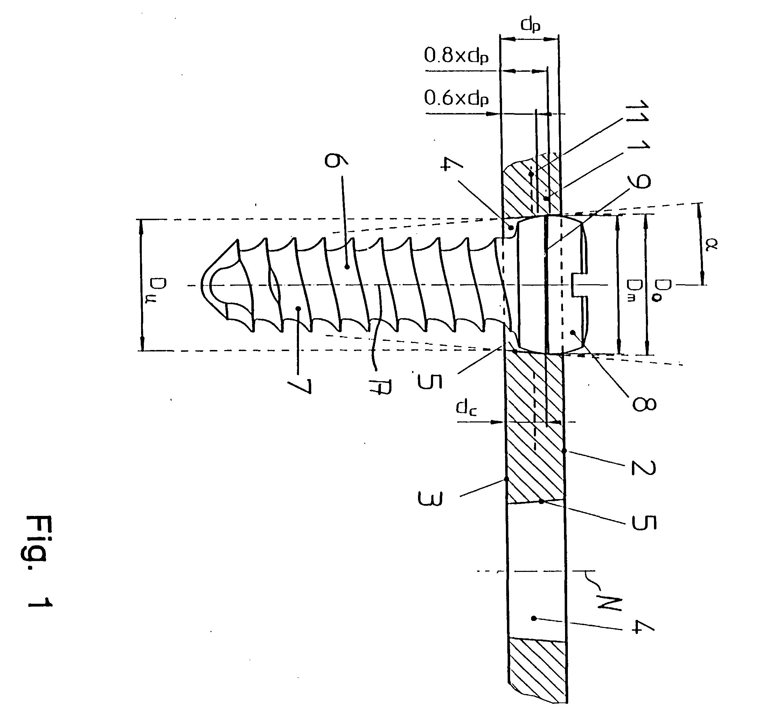

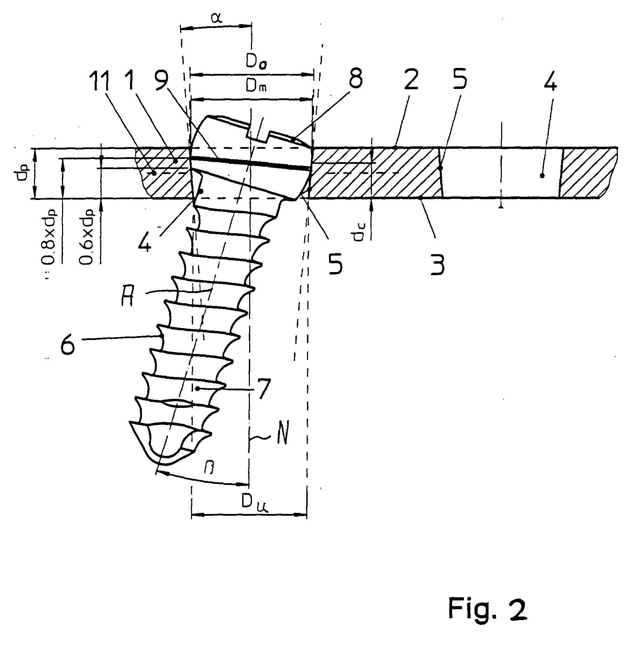

Der maximale Durchmesser Dₘ des Schraubenkopfes 8 ist größer als 1/2 (Dₒ + Dᵤ), so daß die etwa kreisförmige Kontaktfläche 9 mit allen ihren Punkten in der oberen Hälfte der Platte angeordnet ist, jedenfalls dann, wenn die Schraube gemäß Fig. 1 senkrecht montiert ist, d.h. wenn die Längsachse A der Schraube parallel zur Normalen N der Platte 1 verläuft. Wie Fig. 2 zeigt, ist bei diesem bevorzugten Ausführungsbeispiel die Sphärik des Schraubenkopfes 8 so, daß auch bei schräggestellter Schraube 6 die Kontaktfläche 9 mit allen ihren Punkten im wesentlichen über der Mittellinie 11 der Platte 1 liegt.

Bei den dargestellten Ausführungsbeispielen gemäß den Figuren 1 bis 3 ist der Abstand d_{c} der Kontaktfläche 9 von der unteren Oberfläche 3 der Platte zwischen 0,6 dₚ und 0,8 dₚ, und zwar bevorzugt, wie die Figuren 1 und 2 zeigen, für alle möglichen Winkelstellungen der Schraube 6 in Bezug auf die Platte 1.

Die Figuren zeigen auch die konische Form der Schraubenlöcher 4 mit dem Konuswinkel α, der bei den bevorzugten Ausführungsbeispielen zwischen 3° und 5,5°, besonders bevorzugt zwischen 3,5° und 4,5° liegt.

Fig. 2 zeigt die Schraube 6 mit maximal möglichem Neigungswinkel β zwischen der Schraubenachse A und der Normalen N zur Platte 1. Dieser maximale Neigungswinkel ist durch das Anstoßen der Schraube an der Lochkante bestimmt.

Die dargestellte Struktur der Osteosynthesevorrichtung gewährleistet, daß die Schrauben auch bei extremen Zugkräften nicht durch die Löcher 4 rutschen können.

Der Schraubenkopf 8 ist zumindest in den Bereichen, in denen er mit der Wand 5 des Loches in Kontakt kommen kann, aufgerauht. Die rauhe Oberfläche kann zum Beispiel dadurch erhalten werden, daß dieser Bereich des Schraubenkopfes im Anschluß an das Herstellen der Schraube unbehandelt bleibt, also eine gewisse Rauhigkeit beibehält. Die Rauhigkeit kann auch besonders hergestellt werden, insbesondere durch Sandstrahlen oder andere Maßnahmen. Dementsprechend werden bei diesem Ausführungsbeispiel die aufgerauhten Oberflächen nicht eloxiert oder elektrochemisch poliert.

Die Aufrauhung ist so, daß die Reibung, insbesondere die Haftreibung zwischen dem Schraubenkopf und der Schraubenkopf erhöht ist. Auch die Wandung des Schraubenlochs kann entsprechend präpariert werden.

Die angezogene Schraube ergibt eine kreisbandförmige Kontaktfläche, die vollständig im sphärischen Bereich des Schraubenkopfes liegt, und zwar bei allen möglichen zulässigen Relativstellungen zwischen Schraube und Platte (vgl. die in Fig. 2 gezeigte Extremstellung), wobei eine besonders feste, in der Wirkung einer Schweißung vergleichbare Verbindung erreicht wird.

## Patentansprüche

1. Osteosynthesevorrichtung aus einer Platte (1) mit einer oberen Oberfläche (2) und einer gegenüberliegenden, einem Knochen zugekehrten unteren Oberfläche (3) sowie einer Mehrzahl von in der Platte (1) ausgeformten Schraubenlöchern (4), deren Durchmesser (Dₒ) an der oberen Oberfläche (2) der Platte (1) größer ist als ihr Durchmesser (Dᵤ) an der unteren Plattenoberfläche (3), und mit durch die Schraubenlöcher (4) in den Knochen einzuschraubenden Schrauben (6), die einen zumindest teilweise gerundeten Kopf (8) haben, der in eingeschraubtem Zustand mit einer kreisbandförmigen Kontaktfläche (9) an der Wand (5) des Schraubenloches (4) anliegt und sich verkeilt,
**dadurch gekennzeichnet, daß**
zumindest bei senkrecht zur Platte (1) stehender Schraube (6) die Kontaktfläche (9) näher an der oberen als an der unteren Oberfläche der Platte (1) liegt.

2. Osteosynthesevorrichtung nach Anspruch 1,
**dadurch gekennzeichnet, daß**
der gerundete Schraubenkopf (8) zumindest teilweise ballig, insbesondere sphärisch ist.

3. Osteosynthesevorrichtung nach einem der Ansprüche 1 oder 2,
**dadurch gekennzeichnet, daß**
die Rundung des Schraubenkopfes (8) sich soweit in Richtung der Schraubenachse (A) erstreckt, daß bei maximaler Neigung (β) der Schraube (6) gegen die Plattennormale (N) noch eine zur Rundung gehörende bandflächenartige Kontaktfläche (9) voll mit der Wand (5) des Schraubenloches (4) verkeilend in Berührung steht.

4. Osteosynthesevorrichtung nach einem der Ansprüche 2 oder 3,
**dadurch gekennzeichnet, daß**
der Konuswinkel (α) im Bereich von 3° bis 5,5° liegt.

5. Osteosynthesevorrichtung nach Anspruch 4,
**dadurch gekennzeichnet, daß**
der Konuswinkel (α) im Bereich von 3,5° bis 4,5° liegt.

6. Osteosynthesevorrichtung nach einem der vorhergehenden Ansprüche,
**dadurch gekennzeichnet, daß**
zumindest bei senkrecht zur Platte (1) stehender Schraube (6) der Abstand (d_{c}) der Kontaktfläche (9) von der unteren Oberfläche (3) der Platte (1) im Bereich von 0,6 bis 0,8 der Plattenstärke (dₚ) am Lochrand liegt.

7. Osteosynthesevorrichtung nach einem der vorhergehenden Ansprüche,
**dadurch gekennzeichnet, daß**
der Schraubenkopf (8) in eingeschraubtem Zustand in dem Schraubenloch (4) versenkt ist.

8. Osteosynthesevorrichtung nach einem der vorhergehenden Ansprüche,
**dadurch gekennzeichnet, daß**
auch bei im Winkel zur Platte (1) stehender Schraube (6) die Kontaktfläche (9) überall näher an der oberen als an der unteren Oberfläche der Platte (1) liegt.

## Claims

1. An osteosynthesis device comprising a plate (1) having a top face (2) and a bottom face (3), which is located opposite said top face and is directed towards a bone, and a plurality of screw holes (4) which are made in the plate (1) and of which the diameter (Dₒ) at the top face (2) of the plate (1) is larger than the diameter (Dᵤ) at the bottom face (3), and having screws (6) to be screwed into the bone through the screw holes (4) and having an at least partially rounded head (8) which, in the screwed-in state, rests against the wall (5) of the screw hole (4), and wedges there, with an annular contact surface (9),
**characterised in that**, at least when the screw (6) is located perpendicularly with respect to the plate (1), the contact surface (9) is closer to the top face of the plate (1) than to the bottom face thereof.

2. The osteosynthesis device according to Claim 1,
**characterised in that** the rounded screw head (8) is at least partially convex, in particular spherical

3. The osteosynthesis device according to one of Claims 1 or 2,
**characterised in that** the rounding of the screw head (8) extends so far in the direction of the screw axis (A) that, with maximum inclination (β) of the screw (6) with respect to plate normal (N), an annular contact surface (9) belonging to the rounding is still in full wedging contact with the wall (5) of the screw hole (4).

4. The osteosynthesis device according to one of Claims 2 or 3,
**characterised in that** the cone angle ( ) is in the range of from 3° to 5.5°.

5. The osteosynthesis device according to Claim 4,
**characterised in that** the cone angle ( ) is in the range of from 3.5° to 4.5°.

6. The osteosynthesis device according to one of the preceding claims,
**characterised in that**, at least when the screw (6) is located perpendicularly with respect to the plate (1), the distance (d_{c}) of the contact surface (9) from the bottom face (3) of the plate (1) is in the range of from 0.6 to 0.8 of the plate thickness (dₚ) at the edge of the hole.

7. The osteosynthesis device according to one of the preceding claims,
**characterised in that**, in the screwed-in state, the screw head (8) is countersunk in the screw hole (4).

8. The osteosynthesis device according to one of the preceding claims,
**characterised in that**, even when the screw (6) is located at an angle with respect to the plate (1), all points of the contact surface (9) are closer to the top face of the plate (1) than to the bottom face thereof.

## Revendications

1. Dispositif d'ostéosynthèse formé par une plaque (1) possédant une surface supérieure (2) et une surface inférieure opposée (3), tournée vers un os, ainsi qu'une multiplicité de trous de vis (4), qui sont formés dans la plaque (1) et dont le diamètre (Do) au niveau de la surface supérieure (2) de la plaque (1) est supérieur au diamètre (Dᵤ) au niveau de la surface inférieure (3) de la plaque, et comportant des vis (6) pouvant être vissées, à travers les trous de vis (4), dans l'os et qui possèdent une tête (8) au moins partiellement arrondie et qui, à l'état vissé, s'applique par une surface de contact en forme de bande circulaire (9) contre la paroi (5) du trou de vis (4) et s'y coince, **caractérisé en ce que** au moins dans le cas où la vis (6) est perpendiculaire à la plaque (1), la surface de contact (9) est plus près de la surface supérieure que de la surface inférieure de la plaque (1).

2. Dispositif d'ostéosynthèse selon la revendication 1, **caractérisé en ce que** la tête de vis arrondie (8) est au moins partiellement bombée, notamment sphérique.

3. Dispositif d'ostéosynthèse selon l'une des revendications 1 ou 2, **caractérisé en ce que** l'arrondi de la tête de vis (8) s'étend dans la direction de l'axe (A) de la vis au point que dans le cas d'une inclinaison maximale (β) de la vis (6) par rapport à la normale (N) à la plaque, une surface de contact (9), qui fait partie de l'arrondi et correspond à une surface en forme de bande, est complètement en contact avec la paroi (5) du trou de vis (13), avec un effet de coincement.

4. Dispositif d'ostéosynthèse selon l'une des revendications 2 ou 3, **caractérisé en ce que** l'angle (α) du cône se situe dans la gamme comprise entre 3° et 5,5°.

5. Dispositif d'ostéosynthèse selon la revendication 4, **caractérisé en ce que** l'angle (α) du cône se situe dans la gamme comprise entre 3,5° et 4,5°.

6. Dispositif d'ostéosynthèse selon l'une des revendications précédentes, **caractérisé en ce qu'**au moins lorsque la vis (6) est perpendiculaire à la plaque (1), la distance (d_{c}) entre la surface de contact (9) et la surface inférieure (3) de la plaque (1) se situe dans la gamme comprise entre 0,6 et 0,8 fois l'épaisseur (dp) de la plaque au niveau du bord du trou.

7. Dispositif d'ostéosynthèse selon l'une des revendications précédentes, **caractérisé en ce que** la tête de vis (8) est placée en renfoncement, à l'état vissé dans le trou de vis (4).

8. Dispositif d'ostéosynthèse selon l'une des revendications précédentes, **caractérisé en ce que** dans le cas où la vis (6) fait un angle par rapport à la plaque (1), la surface de contact est partout plus proche de la surface supérieure que de la surface inférieure de la plaque (1).
